# EUROPÄISCHE PATENTSCHRIFT

(11) **EP 0 743 301 B1**
(45) Veröffentlichungstag und Bekanntmachung des Hinweises auf die Patenterteilung: **29.08.2001**
(21) Anmeldenummer: 96106881.4
(22) Anmeldetag: 02.05.1996
(51) Int. Cl.: C07C 279/22, C07C 317/32, A61K 31/16, A61K 31/155

(54) **Fluorhaltige Benzoylguanidine**
Benzoylguanidines containing fluorine
Benzoylguanidines contenant du fluor

(30) Priorität: 16.05.1995 DE 19517848
(43) Veröffentlichungstag der Anmeldung: 20.11.1996
(73) Patentinhaber: MERCK PATENT GmbH, 64293 Darmstadt (DE)
(72) Erfinder: Dorsch, Dieter, Dr., 64372 Ober-Ramstadt (DE); Baumgarth, Manfred, Dr., 64297 Darmstadt (DE); Gericke, Rolf, Dr., 64342 Seeheim (DE); Minck, Klaus-Otto, Dr., 64372 Ober-Ramstadt (DE); Beier, Norbert, Dr., 64354 Reinheim (DE)

(56) Entgegenhaltungen:
- EP-A- 0 416 499
- EP-A- 0 556 674
- EP-A- 0 589 336
- EP-A- 0 602 522
- EP-A- 0 602 523
- EP-A- 0 640 588
- EP-A- 0 686 627
- EP-A- 0 699 663
- DE-A- 4 318 756

## Beschreibung

Die Erfindung betrifft fluorhaltige Benzoylguanidine der Formel I 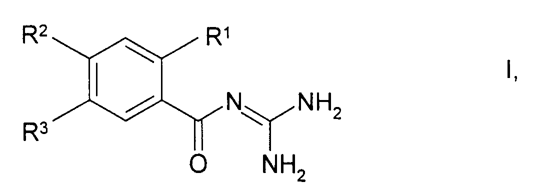 worin
- R¹: CH₃,
- R²: CF₃ oder OCF₃,
- R³: SO₂CH₃,
bedeuten,
sowie deren physiologisch unbedenklichen Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, dass die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d. h. um Wirkstoffe, die den Na⁺/H⁺-Austauschmechanismus der Zellen hemmen (Düsing et al., Med. Klin, 87, 378-384 (1992)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erste Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Darüber hinaus kennt man strukturell ähnliche Verbindungen beispielsweise aus EP 0 416 499, EP 0 699 663 oder EP 0 556 673.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so dass die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zellproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na⁺/H⁺-Antiporters z. B. in Erythrozyten, Thrombozyten oder Leukozyten begleitet werden.

Die Wirkungen der Verbindungen können mit Hilfe an sich bekannter Methoden ermittelt werden, wie sie z. B. von N. Escobales and J. Figueroa in J. Membrane Biol. 120, 41-49 (1991) oder von L. Counillon, W. Scholz, H. J. Lang und J. Pouyssegur in Mol. Pharmacol. 44, 1041-1045 (1993) angegeben werden.

Als Versuchstiere eignen sich z. B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe Verwendung finden.

Gegenstand der Erfindung ist ferner ein Verfahren zur Herstellung der Verbindungen der Formel I gemäß Anspruch 1, sowie von deren Salzen, dadurch gekennzeichnet, dass man eine Verbindung der Formel II 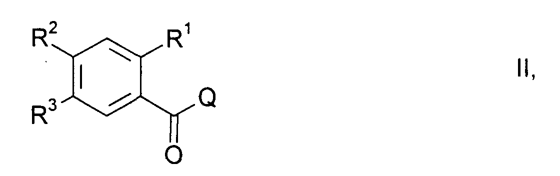 worin
- R¹, R² und R³: die zuvor angegebenen Bedeutungen haben
und
- Q: Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
und/oder dass man eine erhaltene Base der Formel I durch Behandeln mit einer Säure in eines ihrer Salze umwandelt.

Die Verbindungen der Formel I werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

Vorzugsweise werden Verbindungen der Formel I hergestellt, indem man ein aktiviertes Carbonsäurederivat der Formel II, wobei Q besonders bevorzugt Cl oder -O-CH₃ ist, mit Guanidin umsetzt. Besonders geeignet sind Reaktionsvarianten, bei denen die freie Carbonsäure II (Q = OH) in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol, Dicyclohexylcarbodiimid oder die Mukaiyama-Variante (Angew. Chem. 91, 788-812 (1979)).

Die Umsetzung eines reaktionsfähigen Carbonsäurederivates der Formel II mit Guanidin erfolgt in an sich bekannter Weise vorzugsweise in einem protischen oder aprotischen polaren oder unpolaren inerten organischen Lösungsmittel.

Bei der Herstellung von II, bei der Umsetzung von II mit Guanidin ist es zweckmäßig, in Gegenwart einer Base oder mit einem Überschuss der basischen Komponente zu arbeiten. Als Basen eignen sich bevorzugt z. B.

Alkalimetall- oder Erdalkalimetallhydroxide, -carbonate, -alkoholate oder organische Basen wie Trieethylamin oder Pyridin, die auch im Überschuss angewendet werden und dann gleichzeitig als Lösungsmittel dienen können.

Als inerte Lösungsmittel eignen sich insbesondere Alkohole wie Methanol, Ethanol, Isopropanol, n-Butanol oder tert.-Butanol, Ether wie Diethylether, Diisopropylether, Tetrahydrofuran (THF) oder Dioxan, Glykolether wie Ethylenglykolmonomethyl- oder -monoethylether (Methylglykol oder Ethylglykol), Ethylenglykoldimethylether (Diglyme), Ketone wie Aceton oder Butanon, Nitrile wie Acetonitril, Nitroverbindungen wie Nitromethan oder Nitrobenzol, Ester wie Ethylacetat, Amide wie Phosphorsäurehexamethyltriamid, Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol oder Xylol. Weiterhin eignen sich Gemische dieser Lösungsmittel untereinander.

Eine Base der Formel I kann mit einer Säure in das zugehörige Säureadditionssalz übergeführt werden. Für diese Umsetzung kommen Säuren in Frage, die physiologische unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxethansulfonsäure, Benzolsulfonsäure, p-Toluolsäure, Naphthalinmono- und Disulfonsäuren, Laurylschwefelsäure.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfssstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eine ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z. B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z. B. zur Herstellung von Injektionspräparaten verwendet werden.

Insbesondere für die topische Anwendung kommen auch liposomale Zubereitungen in Betracht. Die angegebenen Zubereitungen können sterilisiert sein und/oder Hilfsstoffe wie Geleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tieren, insbesondere Säuretiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden von Angina pectoris, Infarkten, Ischämien des Nervensystems wie z. B. Schlaganfall oder Hirnödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankungen, Fibrosen sowie Organhypertrophien und -hyperplasien.

Dabei werden die erfindungsgemäßen Substanzen in der Regel in Analogie zu bekannten Antiarrhythmika, z. B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

Man rührt eine Lösung von 0,5 g 2-Methyl-4-phenoxy-5-trifluormethylbenzoesäure [erhältlich durch Umsetzung von 2-Methyl-4-phenoxy-5-brom-benzoesäuremethylester mit Kaliumtrifluoracetat in Gegenwart von Cul und Tetramethylammoniumiodid und anschließende Verseifung] und 300 mg Carbonyldiimidazol in 15 ml THF zwei Stunden bei Raumtemperatur und fügt diese Anschließend zu 383 mg Guanidin. Man rührt weitere zwei Stunden. Nach üblicher Aufarbeitung erhält man das N-Diaminomethylen-2-methyl-4-phenoxy-5-trifluormethyl-benzamid.

Analog erhält man durch Umsetzung von Guanidin
- mit: 2-Methyl-4-trifluormethoxy-5-methylsulfonyf-benzoesäure das N-Diaminomethylen-2-methyl-4-trifluormethoxy-5-methylsulfonylbenzamid.

### Beispiel 2

Zu einer Lösung von 928 mg Guanidin in 15 ml Methanol werden 1,1 g 3-Methylsulfonyl-4-trifluormethyl-benzoesäuremethylester [F. 146-147° ; erhältlich durch Umsetzung von 3-Methylsulfonyl-4-brom-benzoesäuremethylester mit Kalium-trifluoracetat in Gegenwart von Cul und Tetramethylammonium-iodid in Toluol] hinzugefügt. Man rührt 45 Minuten bei 50° und erhält nach Entfernung des Lösungsmittels und üblicher Aufarbeitung das N-Diamino-methylen-3-methylsulfonyl-4-trifluormethylbenzamid, F. 233-234°.

Nach Behandlung mit verdünnter wässriger HCI-Lösung und Gefriertrocknung wird daraus das entsprechende Hydrochlorid erhalten.

Analog erhält man durch Umsetzung von Guanidin
- mit: 2-Methyl-4-trifluormethyl-5-methylsulfonyl-benzoesäuremethylester (F. 135-136°) das N-Diaminomethylen-2-methyl-4-trifluormethyl-5-methylsulfonylbenzamid, F. 212-213° (Base), Hydrochlorid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injketionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und lässt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann beispielsweise in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel F: Dragees

Analog Beispiel E werden Tabletten gepresst, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so dass jede Kapsel 20 mg des Wirkstoffs enthält.

### Beispiel H. Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Fluorhaltige Benzoylguanidine der Formel I 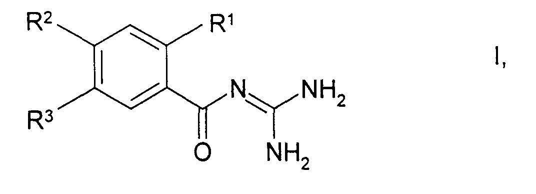 worin
R¹ CH₃,
R² CF₃ oder OCF₃,
R³ SO₂CH₃,
bedeuten,
sowie deren physiologisch unbedenklichen Salze.

2. N-Diaminomethylen-2-methyl-Verbindungen gemäß Anspruch 1
(a) N-Diaminomethylen-2-methyl-4-trifluormethoxy-5-methylsulfonylbenzamid;
(b) N-Diaminomethylen-2-methyl-4-trifluormethyl-5-methylsulfonyl-benzamid
sowie deren unbedenklichen Salze.

3. Verfahren zur Herstellung von fluorhaltigen Benzoylguanidin-Derivaten der Formel I gemäß Anspruch 1 sowie von deren Salzen, **dadurch gekennzeichnet**, dass man eine Verbindung der Formel II 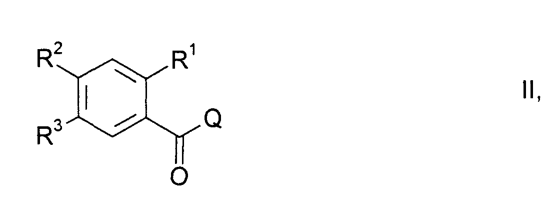 worin
R¹, R² und R³ die zuvor angegebenen Bedeutungen haben
und
Q Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
und/oder dass man eine erhaltene Base der Formel I durch Behandeln mit einer Säure in eines ihrer Salze umwandelt.

4. Verfahren zur Herstellung pharmazeutischer Zubereitungen, **dadurch gekennzeichnet**, dass man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

7. Verwendung von Verbindungen der Formel I nach Anspruch 1 zur Herstellung von Arzneimitteln zur Behandlung von Arrhythmien, Angina pectoris, Infarkten sowie zur präventiven Behandlung der genannten Indikationen.

## Claims

1. Fluorine-containing benzoylguanidines of the formula I 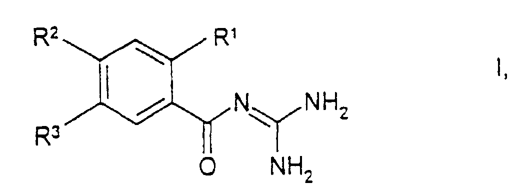 in which
R¹ is CH₃,
R² is CF₃ or OCF₃,
R³ is SO₂CH₃,
and their physiologically acceptable salts.

2. N-Diaminomethylene-2-methyl compounds according to Claim 1
(a) N-diaminomethylene-2-methyl-4-trifluoromethoxy-5-methylsulfonylbenzamide;
(b) N-diaminomethylene-2-methyl-4-trifluoromethyl-5-methylsulfonylbenzamide;
and their physiologically acceptable salts.

3. Process for the preparation of fluorine-containing benzoylguanidine derivatives of the formula I according to Claim 1 and of their salts, **characterized in that** a compound of the formula II 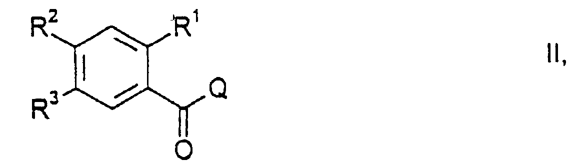 in which
R¹, R² and R³ have the meanings indicated above
and
Q is Cl, Br, OA, O-CO-A, O-CO-Ph, OH or another reactive esterified OH group or easily nucleophilically substitutable leaving group,
is reacted with guanidine,
and/or in that a base of the formula I which is obtained is converted into one of its salts by treating with an acid.

4. Process for the production of pharmaceutical preparations, **characterized in that** a compound of the formula I according to Claim 1 and/or one of its physiologically acceptable salts is brought into a suitable dose form together with at least one solid, liquid or semi-liquid excipient or auxiliary.

5. Pharmaceutical preparation, **characterized in that** it contains at least one compound of the general formula I according to Claim 1 and/or one of its physiologically acceptable salts.

6. Use of compounds of the formula I according to Claim 1 or of their physiologically acceptable salts for the production of a medicament.

7. Use of compounds of the formula I according to Claim 1 for the production of medicaments for the treatment of arrhythmias, angina pectoris, infarcts and also for the preventive treatment of the indications mentioned.

## Revendications

1. Benzoylguanidines fluorées de formule I 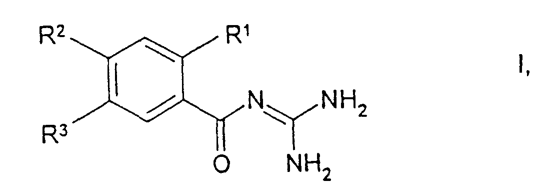 dans laquelle
R¹ représente CH₃,
R² représente CF₃ ou OCF₃,
R³ représente SO₂CH₃,
ainsi que leurs sels physiologiquement acceptables.

2. Composés N-diaminométhylène-2-méthyle selon la revendication 1
(a) N-diaminométhylène-2-méthyl-4-trifluorométhoxy-5-méthylsulfonylbenzamide,
(b) N-diaminométhylène-2-méthyl-4-trifluorométhyl-5-méthylsulfonylbenzamide
ainsi que leurs sels physiologiquement acceptables.

3. Procédé pour la préparation de dérivés de type benzoylguanidine fluorée de formule 1 selon la revendication 1 ainsi que de leurs sels, **caractérisé en ce que** l'on fait réagir avec de la guanidine un composé de formule II 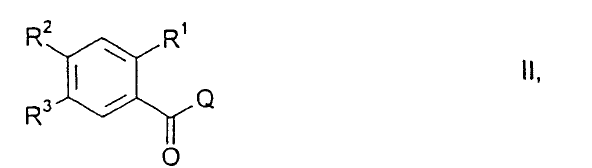 dans laquelle R¹, R² et R³ ont les significations indiquées précédemment, et
Q représente Cl, Br, OA, O-CO-A, O-CO-Ph, OH ou un autre groupe OH estérifié réactif, ou un groupe partant aisément remplaçable par substitution nucléophile,
et/ou en ce que l'on convertit en un de ses sels une base obtenue de formule I, par traitement par un acide.

4. Procédé pour la préparation de compositions pharmaceutiques, **caractérisé en ce que** l'on met sous une forme galénique appropriée un composé de formule I selon la revendication 1 et/ou un de ses sels physiologiquement acceptables, conjointement avec au moins un véhicule ou adjuvant solide, liquide ou semi-liquide.

5. Composition pharmaceutique, caractérisée par une teneur en au moins un composé de formule générale I selon la revendication 1 et/ou un de ses sels physiologiquement acceptables.

6. Utilisation des composés de formule I selon la revendication 1 ou de leurs sels physiologiquement acceptables, pour la fabrication d'un médicament.

7. Utilisation des composés de formule I selon la revendication 1 pour la fabrication de médicaments destinés au traitement d'arythmies, de l'angine de poitrine, d'infarctus, ainsi qu'au traitement préventif des indications citées.
